# EUROPÄISCHE PATENTSCHRIFT

(11) **EP 1 452 500 B1**
(45) Veröffentlichungstag und Bekanntmachung des Hinweises auf die Patenterteilung: **11.05.2011**
(21) Anmeldenummer: 03027700.8
(22) Anmeldetag: 04.12.2003
(51) Int. Cl.: C03C 14/00, A61K 6/06

(54) **Glaskeramik sowie deren Herstellung und Verwendung**
Glass-ceramic, and its production and use
Vitrocéramique, et sa production et utilisation

(30) Priorität: 27.02.2003 DE 10310001
(43) Veröffentlichungstag der Anmeldung: 01.09.2004
(73) Patentinhaber: Wieland Dental Ceramics GmbH, 61191 Rosbach-Rodheim (DE)
(72) Erfinder: Assmann, Steffen, 61169 Friedberg (DE); Appel, Peter, 61200 Wölfersheim (DE); Armbrust, Reinhard, 61118 Bad Vilbl (DE)
(74) Vertreter: Patentanwälte Ruff, Wilhelm, Beier, Dauster & Partner

(56) Entgegenhaltungen:
- EP-A- 1 245 548
- EP-A- 1 253 116
- US-A- 5 944 884

## Beschreibung

Die Erfindung betrifft eine Glaskeramik, Verfahren zur Herstellung dieser Glaskeramik sowie ihre Verwendung für dentale Zwecke.

Bei Glaskeramiken handelt es sich bekanntlich um Festkörper, bei denen neben der Glasphase kristalline Bereiche vorliegen. Die Mikrostruktur solcher Glaskeramiken läßt sich auch so beschreiben, daß in eine oder mehrere Glasphasen (die sogenannte Glasmatrix) Kristalle, die die sogenannte Kristallphase bilden, eingelagert sind.

Glaskeramiken werden bereits seit Anfang der sechziger Jahre auch in der Dentaltechnik eingesetzt, insbesondere als sogenannte Verblendkeramiken zur Beschichtung/Verblendung meist metallischer Gerüste. Zum historischen Stand der Technik sei hier auf die beiden US-Patente 3,052,982 und 3,052,983 von Weinstein verwiesen. Bei diesen Anwendungen wird (tetragonales) Leucit als Kristallphase mit einem hohen Wärmeausdehnungskoeffizienten (WAK-Wert, 25 °C bis 500 °C) von ca. 20 x 10⁻⁶/K mit einer Glasphase, die einen niedrigen WAK-Wert von beispielsweise ca. 8 x 10⁻⁶/K aufweist, kombiniert. Durch Auswahl der Mischungsverhältnisse dieser Komponenten können auf diese Weise unterschiedliche Wärmeausdehnungskoeffizienten für die Glaskeramik eingestellt werden.

Die EP 1253116 beschreibt eine pressbare Glaskeramik, die neben synthetischem Leucit einen sehr hohen Anteil (30 - 90 Gew.-%) an Lithium-Silicatglas enthält. Das synthetische Leucit wird dabei durch Vermischen von Kaliumcarbonat, Aluminiumoxid und Silziumdioxid in stöchiometrischem Verhältnis und anschließende Wärmebehandlung der Mischung gewonnen. Das so erhaltene Leucit wird zu einem Pulver mit einer durchschnittlichen Korngröße zwischen 1 und 100 µm gemahlen.

Die EP 1245548 betrifft ein Verfahren zur Herstellung einer leucithaltigen Glaskeramik, nach dem das Leucit nicht als separate Komponente in die Glaskeramik eingebracht wird sondern erst im Verlauf einer Wärmebehandlung gebildet wird. Dazu wird ein glasartiges Material mit einer kleinen Menge an Leucit-Impfkristallen mit einer bevorzugten Größe zwischen 30 und 60 µm versetzt und anschließend wärmebehandelt.

In der US 5,944,884 wird eine für Dentalprodukte geeignete Glaskeramik beschrieben, die Leucit-Kristalle mit einem Durchmesser < 10 µm, vorzugsweise < 5 µm, aufweist. Die Begrenzung der Korngröße soll zu einer gleichmäßigen Oberfläche führen und Irritationen in der Mundhöhle vermeiden. In weiter bevorzugten Ausführungsformen liegt der Durchmesser der Leucitkristalle unter 1 µm. Die Glaskeramiken enthalten Li₂O in einem Anteil von 0,5 - 3 Gew.-%, vorzugsweise von 0,7 - 1,5 Gew.-%.

Der Einsatz in der Dentaltechnik stellt an die Glaskeramiken, gerade auch im Hinblick auf ihre mechanische Belastbarkeit, besondere Anforderungen. So stellt das die Kristallphase (Sekundärphase) bildende Leucit eine potentielle Schwachstelle für Risse oder sogar eine Sollbruchstelle im Werkstoff dar. Dementsprechend ist eine Rißbildung, insbesondere in der Glasphase und auch innerhalb der Kristallphase zu vermeiden. Die bisher realisierten Mikrostrukturen entsprechender Glaskeramiken aus Glasphase und Leucit leisten dies in der Regel nicht.

Zum einen werden in entsprechenden Glaskeramiken Leucitkristalle mit einer Größe bis zu 60 µm eingesetzt. Solche Glaskeramiken weisen aufgrund der großen Differenz der WAK-Werte der Einzelkomponenten und aufgrund der vergleichsweise großen Leucitkristalle häufig sowohl in der Glasphase als auch in der Kristallphase (Leucitphase) Risse auf. Die Leucitphase ist in diesen Glaskeramiken heterogen in der Glasphase/ Glasmatrix verteilt.

Bei einer anderen bekannten Gruppe von Glaskeramiken werden kleinere Leucitkristalle eingesetzt. So beschreibt die EP 0 690 030 Glaskeramiken, die neben einer Leucitphase noch eine Fluoroapatitphase als weitere Kristallphase enthalten, wobei die enthaltenen Leucitkristalle mittlere Korngrößen < 5 µm aufweisen sollen. Auch die US 5,653,791 beschreibt Glaskeramiken mit bestimmten chemischen Zusammensetzzungen, die Leucitkristalle enthalten, deren Korngrößen 10 µm nicht überschreiten.

Auch Glaskeramiken mit kleineren Leucitkristallen zeigen jedoch häufig eine große Neigung zur Rißbildung (sowohl in der Glasphase als auch in der Leucitphase) mit Rissen in der Größenordnung von bis zu 1 µm. Zudem neigen die Leucitkristalle vieler dieser Keramiken im Fall von Mehrfachbränden zu einem unkontrollierten Kristallwachstum, in der Folge treten wieder Spannungen und Risse auf.

Die Erfindung stellt sich dementsprechend die Aufgabe, die geschilderten Nachteile der Glaskeramiken des Standes der Technik zu vermeiden. So soll die Rißbildung in der Glaskeramik weitgehend unterdrückt und möglichst sogar vollständig vermieden werden. Durch eine weitgehend optimierte Mikrostruktur sollen die entsprechenden Glaskeramiken in besonderer Weise für den Einsatz im Dentalbereich geeignet sein.

Diese Aufgabe wird gelöst durch die Glaskeramik mit den Merkmalen des Anspruchs 1 und das Verfahren mit den Merkmalen des Anspruchs 9. Bevorzugte Ausführungen dieser Glaskeramik und dieses Verfahrens sind in den abhängigen Ansprüchen 2 bis 8 bzw. 10 bis 12 beschrieben. Die Ansprüche 13 bis 15 definieren eine erfindungsgemäße Verwendung der beanspruchten Glaskeramik bzw. einen entsprechenden Zahnersatz. Der Wortlaut sämtlicher Ansprüche wird hiermit durch Bezugnahme zum Inhalt dieser Beschreibung gemacht.

Die erfindungsgemäße Glaskeramik besitzt eine kontinuierliche Glasphase und eine Kristallphase aus tetragonalem Leucit. Dabei ist die Glasphase rißfrei (nach gängigem Verständnis bei mikroskopischer Betrachtung), und die Kristallphase ist in dieser Glasphase im wesentlichen homogen verteilt. Die Leucitkristalle in der Kristallphase weisen erfindungsgemäß die unten definierte Korngrößenverteilung auf, mit Korngrößen < 1 µm und Korngrößen ≥ 1 µm.

lm Gegensatz zum Stand der Technik sind also nach der Erfindung Leucitkristalle in der Glaskeramik vorhanden, die Korngrößen < 1 µm besitzen und folglich im Nanometer-Größenbereich liegen. Die Mikrostruktur der beanspruchten Glaskeramik kann somit auch als "Nanoleucitstruktur" bezeichnet werden. Die insgesamt vorhandenen Leucitkristalle sind in der rißfreien Glasphase/Glasmatrix im wesentlichen homogen verteilt. Unter dieser "im wesentlichen homogenen Verteilung" soll verstanden werden, daß sich bei einer mikroskopischen/elektronenmikroskopischen Betrachtung in gleich großen Bereichen der Glasphase im Mittel ungefähr gleich viel Leucitkristalle befinden.

Aus Gründen der Vollständigkeit sei erwähnt, daß Leucit durch die chemische Formel K[AlSi₂O₆] dargestellt wird und erfindungsgemäß in seiner tetragonalen Struktur eingesetzt wird.

Bezüglich der eingesetzten Gläser/Glasmatrix ist die Erfindung grundsätzlich nicht beschränkt. Besonders bevorzugt lassen sich erfindungsgemäß Silikatgläser einsetzen, wobei hier insbesondere Gläser mit nennenswerten Anteilen an Alkalimetallionen, also sogenannte Alkalisilikatgläser, hervorzuheben sind.

Bei allen vorhergehenden und noch folgenden Angaben sind die Gew.-%-Werte auf die Glaskeramik insgesamt bezogen.

Die Glaskeramiken nach der Erfindung enthalten die Komponenten
- 60 Gew.-% bis 70 Gew.-% SiO₂,
- 10 Gew.-% bis 15 Gew.-% Al₂O₃,
- 10 Gew.-% bis 15 Gew.-% K₂O,
- 2 Gew.-% bis 7 Gew.-% Na₂O,
- 0 Gew.-% bis 0,3 Gew.-% Li₂O,
- 0,1 Gew.-% bis 0,5 Gew.-% Sb₂O₃,
- 0,1 Gew.-% bis 0,5 Gew.-% BaO,
- 0,5 Gew.-% bis 1,0 Gew.-% CaO,
- 0,1 Gew.-% bis 0,4 Gew.-% F.

Von diesen sind wiederum solche Glaskeramiken bevorzugt, die die folgenden Komponenten enthalten:
- 63 Gew.-% bis 67 Gew.-% SiO₂,
- 12 Gew.-% bis 15 Gew.-% Al₂O₃,
- 10 Gew.-% bis 14 Gew.-% K₂O,
- 2 Gew.-% bis 6,5 Gew.-% Na₂O,
- 0,1 Gew.-% bis 0,2 Gew.-% Li₂O,
- 0,1 Gew.-% bis 0,3 Gew.-% Sb₂O₃,
- 0,1 Gew.-% bis 0,3 Gew.-% BaO,
- 0,6 Gew.-% bis 1,0 Gew.-% CaO,
- 0,1 Gew.-% bis 0,3 Gew.-% F.

Bei allen Ausführungsformen der erfindungsgemäßen Glaskeramik ist es möglich, daß der Glaskeramik noch Zusatzstoffe, bevorzugt Farbstoffe, insbesondere Farbpigmente, in vorzugsweise geringen Mengen zugesetzt sind. Der Zusatz solcher Farbpigmente ist insbesondere bei der später noch angesprochenen Verwendung der Glaskeramik im Dentalbereich angezeigt, beispielsweise um die verschiedenen Farbtöne von Verblendkeramiken zu realisieren.

Die Leucitkristalle mit Korngrößen < 1 µm, die bei der Erfindung als "erste Gruppe" von Kristallen bezeichnet werden sollen, sind bei den beanspruchten Glaskeramiken zwischen ca. 5 % bis ca. 50 %, bezogen auf 100 % der insgesamt vorhandenen Leucitkristalle, vorhanden. Innerhalb dieses Bereichs ist es bevorzugt, wenn ca. 5 % bis ca. 35 %, insbesondere ca. 5 % bis ca. 20 % Leucitkristalle der ersten Gruppe vorhanden sind.

In weiter bevorzugten Ausführungen der erfindungsgemäßen Glaskeramik besitzen die Leucitkristalle der ersten Gruppe Korngrößen von weniger als 0,5 µm, insbesondere von weniger als 0,3 µm.

Die erfindungsgemäß vorhandenen Leucitkristalle mit Korngrößen ≥ 1 µm sollen bei der Erfindung als "zweite Gruppe" von Kristallen bezeichnet werden. Die Anteile der Leucitkristalle der zweiten Gruppe ergeben sich entsprechend aus den obigen Angaben für die Anteile der Leucitkristalle der ersten Gruppe. Die Leucitkristalle der zweiten Gruppe besitzen nach der Erfindung vorzugsweise Korngrößen zwischen 1 µm und 10 µm, wobei innerhalb dieses Bereiches Korngrößen von 1 µm bis 7 µm weiter bevorzugt sind.

In Übereinstimmung mit den bisherigen Ausführungen besitzt die erfindungsgemäße Glaskeramik eine bestimmte Korngrößenverteilung der Leucitkristalle in der Kristallphase, derart, daß
- ca. 5 % bis ca. 50 % der Leucitkristalle Korngrößen < 1 µm aufweisen (Leucitkristalle der ersten Gruppe),
- bis zu ca. 1 %, vorzugsweise bis zu ca. 0,5 % der Leucitkristalle Korngrößen > 7 µm aufweisen (erster Anteil der Leucitkristalle der zweiten Gruppe), und
- der Rest, bezogen auf 100 % der insgesamt vorhandenen Leucitkristalle, Leucitkristalle mit Korngrößen zwischen 1 µm und 7 µm (zweiter Anteil der Leucitkristalle der zweiten Gruppe) aufweist.

Erfindungsgemäß ist die beanspruchte Glaskeramik bei besonderen Ausführungsformen weiter dadurch gekennzeichnet, daß (nicht nur die Glasphase) auch die Kristallphase (Leucitphase) im wesentlichen frei von Rissen ist.

Die erfindungsgemäße Glaskeramik weist vorzugsweise einen Wärmeausdehnungskoeffizienten (WAK-Wert, 25 °C bis 500 °C) zwischen 11 und 16,5 x 10⁻⁶/K auf sowie eine bevorzugte Brenntemperatur zwischen 700 °C und 950 °C.

Das erfindungsgemäße Verfahren zur Herstellung der beanspruchten Glaskeramik ist dadurch gekennzeichnet, daß die die Glasphase/Glasmatrix bildenden Glaspartikel, beispielsweise mit d₅₀-Werten zwischen 2 µm und 20 µm, und Leucitkristalle mit entsprechender Korngrößenverteilung miteinander vermischt werden. Dann wird die so erhaltene Mischung einer Wärmebehandlung (Tempern) bei Temperaturen zwischen 700 °C und 1.100 °C unterzogen. Innerhalb dieses Temperaturbereiches sind für die Wärmebehandlung Temperaturen zwischen 850 °C und 1.050 °C bevorzugt.

Die Dauer der beschriebenen Wärmebehandlung kann grundsätzlich frei gewählt werden. Es ist jedoch bevorzugt, die Wärmebehandlung in Zeiträumen zwischen 10 min. und 2 Stunden, vorzugsweise zwischen 30 min. und 1,5 Stunden, vorzunehmen. In vielen Fällen ist es weiter bevorzugt, wenn die Wärmebehandlung über einen Zeitraum von ca. 1 Stunde durchgeführt wird.

Leucit, wie er bei dem erfindungsgemäßen Verfahren eingesetzt werden kann, ist kommerziell erhältlich. Er kann jedoch auch stöchiometrisch gemäß der oben dargestellten chemischen Formel eingewogen und erschmolzen werden.

Ein besonders bevorzugtes erfindungsgemäßes Verfahren umfaßt die Herstellung der Leucitkristalle wie folgt:
- stöchiometrisches Einwiegen der Komponenten für den Leucit, vorzugsweise Kaliumoxid (K₂O), Aluminiumoxid (Al₂O₃) und Siliziumdioxid (SiO₂),
- Erschmelzen der erhaltenen stöchiometrischen Mischung bei Temperaturen zwischen 1.400 °C und 1.600 °C,
- Wärmebehandlung/Tempern des erschmolzenen Produkts, vorzugsweise bei einer Temperatur von ca. 1.000 °C für 1 Stunde,
- Zerkleinern des wärmebehandelten/getemperten Produkts auf die gewünschte Korngrößenverteilung, vorzugsweise durch mindestens einen Mahlvorgang.

Selbstverständlich kann die erwünschte Korngrößenverteilung auch durch Zusammenmischen entsprechender Fraktionen von Leucitkristallen erhalten werden.

Wie bereits erwähnt, werden durch die erfindungsgemäße Glaskeramik und durch das erfindungsgemäße Verfahren die eingangs geschilderten Nachteile des Standes der Technik vermieden. Durch die neu definierte Mikrostruktur mit der homogenen und feinverteilten Kristallphase/Leucitphase ist die Rißbildung in der Glaskeramik minimiert. In der Glasphase treten dabei gar keine, in der Leucitphase, wenn überhaupt, nur äußerst vereinzelt Risse auf. Aufgrund dieser Mikrostruktur besitzt die erfindungsgemäße Glaskeramik sehr gute Werkstoffeigenschaften, die sich auch in einer glatten Oberfläche sowie einer sehr guten Polierbarkeit des Materials bemerkbar machen.

An dieser Stelle soll auch nochmals der vorzugsweise geringe Anteil an Li₂O in der Glaskeramik hervorgehoben werden. Überraschenderweise wurde festgestellt, daß bei Li₂O-Anteilen < 0,3 Gew.-% das bei Mehrfachbränden häufig auftretende unkontrollierte Wachstum der Leucitkristalle stark eingeschränkt ist. Durch unkontrolliertes Wachstum können in der Keramik Spannungen auftreten, die in der Folge wieder zu Rissen sowohl in der Glasmatrix, als auch in den Kristallen selbst führen. Bei niedrigem Li₂O-Gehalt bleiben die Kristalle in der Matrix stabil.

Alle diese Eigenschaften machen die erfindungsgemäße Keramik für die Anwendung im Dentalbereich besonders geeignet.

Dementsprechend umfaßt die Erfindung auch die Verwendung der beanspruchten Glaskeramik für dentale Zwecke, insbesondere als Dentalmaterial. Eine besonders bevorzugte Verwendung der beanspruchten Glaskeramik ist die Verblendung von Zahnersatz, insbesondere für sogenannten metallkeramischen Zahnersatz. Dies sind bekanntlich solche Systeme, bei denen ein Grundgerüst/Grundkörper aus Metallen oder Metallegierungen mit der entsprechenden Glaskeramik (Dentalkeramik) beschichtet bzw. verblendet wird. Die WAK-Werte (25 °C bis 500 °C) der Glaskeramik liegen dabei im Regelfall um 0,5 bis 2 Einheiten unterhalb der WAK-Werte der Gerüstmaterialien. Selbstverständlich ist die beanspruchte Glaskeramik aber beispielsweise auch als Material für sogenannte Inlays, Onlays und Veneers einsetzbar.

Schließlich umfaßt die Erfindung den Zahnersatz selbst, der nach seiner Herstellung eine erfindungsgemäße Glaskeramik aufweist (Anspruch 15). Hier handelt es sich insbesondere um einen sogenannten metallkeramischen Zahnersatz, d. h. in der Regel um einen Grundkörper oder ein Gerüst aus einem Metall oder einer Metallegierung, das mit der beanspruchten Glaskeramik beschichtet und/oder verblendet ist.

Weitere Merkmale der Erfindung ergeben sich aus den nachfolgenden Beispielen in Verbindung mit den Unteransprüchen. Hierbei können die dargestellten Merkmale und Eigenschaften jeweils für sich allein oder zu mehreren in Kombination miteinander verwirklicht sein.

### Leucitkristalle

Zur Herstellung von Leucitkristallen werden K₂O, Al₂O₃ und SiO₂ (Quarz) stöchiometrisch gemäß der chemischen Formel K[AlSi₂O₆] eingewogen und die erhaltene Mischung bei 1.500 °C erschmolzen. Das abgekühlte erschmolzene Produkt wird anschließend einer Wärmebehandlung bei 1.000 °C über einen Zeitraum von 60 min. unterworfen. Dann wird das erhaltene wärmebehandelte Produkt in einer Mühle fein gemahlen, bis die folgende Korngrößenverteilung erhalten wird:
- ca. 20 % Leucitkristalle im Nanometerbereich, d. h. < 1 µm (erste Gruppe der Leucitkristalle),
- ca. 79 % Leucitkristalle im unteren µm-Bereich zwischen 1 µm und 7 µm (größerer Anteil der zweiten Gruppe Leucitkristalle), und
- Rest, ca. 1 %, Leucitkristalle mit Körngrößen > 7 µm (geringerer Anteil der zweiten Gruppe Leucitkristalle).

### Beispiel

Ein Alkalisilikatglas wird aus den folgenden Komponenten bei ca. 1.500 °C erschmolzen:

| | |
|---|---|
| SiO₂ | 77,8 Gew.-% |
| Al₂O₃ | 4,0 Gew.-% |
| K₂O | 3,8 Gew.-% |
| Na₂O | 11,4 Gew.-% |
| Li₂O | 0,2 Gew.-% |
| Sb₂O₃ | 0,4 Gew.-% |
| BaO | 0,4 Gew.-% |
| CaO | 1,6 Gew.-% |
| F | 0,4 Gew.-% |

Das so hergestellte Alkalisilikatglas wird zu feinen Partikeln gemahlen. Anschließend erstellt man eine Mischung, bestehend aus
- 50 Gew.-% Glaspartikel obiger Zusammensetzung,
- 48,5 Gew.-% der oben beschriebenen Leucitkristalle und
- 1,5 Gew.-% Pigmenten.

Diese Mischung wird einer Wärmebehandlung bei ca. 1.000 °C über einen Zeitraum von 60 min. unterzogen. Man erhält eine erfindungsgemäße Glaskeramik mit einer Brenntemperatur von 900 °C und einem WAK-Wert (25 °C bis 500 °C) von ca. 13 × 10⁻⁶/K.

Die Glaskeramik ist in hervorragender Weise als Verblendkeramik für dentale Zwecke geeignet. Sie ist ausgezeichnet mit einem Metallgerüst, beispielsweise aus einer hochgoldhaltigen Legierung mit einem WAK-Wert im Bereich von 13,8 x 10⁻⁶/K bis 15,1 x 10⁻⁶/K, verträglich und leicht im Patientenmund bearbeitbar, beispielsweise durch Polieren.

Die Gesamtzusammensetzung der so hergestellten Glaskeramik wurde durch eine Röntgenfluoreszenzanalyse bestimmt. Sie ergibt sich in ihren Hauptbestandteilen wie folgt:
- 65,6 Gew.-% SiO₂,
- 13,4 Gew.-% Al₂O₃,
- 12,4 Gew.-% K₂O,
- 5,7 Gew.-% Na₂O,
- 0,1 Gew.-% Li₂O,
- 0,2 Gew.-% Sb₂O₃,
- 0,2 Gew.-% BaO,
- 0,8 Gew.-% CaO,
- 0,2 Gew.-% F.

## Patentansprüche

1. Glaskeramik mit einer kontinuierlichen Glasphase und einer Kristallphase aus tetragonalem Leucit, wobei die Glasphase frei von Rissen ist und die Kristallphase aus Leucitkristallen im wesentlichen homogen in der Glasphase verteilt ist und folgende Korngrößenverteilung aufweist:
- ca. 5 % bis ca. 50 % einer ersten Gruppe von Kristallen mit Korngrößen < 1 µm und
- ca. 50 % bis ca. 95 % einer zweiten Gruppe von Kristallen mit Korngrößen ≥ 1 µm,
wobei bis zu ca. 1 % der Kristalle der zweiten Gruppe Korngrößen > 7 µm aufweisen und der Rest, bezogen auf 100 %, Korngrößen zwischen 1 µm und 7 µm aufweist,
und wobei die Glaskeramik die folgenden Komponenten enthält:
- 60 Gew.-% bis 70 Gew.-% SiO₂,
- 10 Gew.-% bis 15 Gew.-% Al₂O₃,
- 10 Gew.-% bis 15 Gew.-% K₂O,
- 2 Gew.-% bis 7 Gew.-% Na₂O,
- 0 Gew.-% bis 0,3 Gew.-% Li₂O,
- 0,1 Gew.-% bis 0,5 Gew.-% Sb₂O₃,
- 0,1 Gew.-% bis 0,5 Gew.-% BaO,
- 0,5 Gew.-% bis 1,0 Gew.-% CaO,
- 0,1 Gew.-% bis 0,4 Gew.-% F.

2. Glaskeramik nach Anspruch 1, **dadurch gekennzeichnet, daß** sie die folgenden Komponenten enthält:
- 63 Gew.-% bis 67 Gew.-% SiO₂,
- 12 Gew.-% bis 15 Gew.-% Al₂O₃,
- 10 Gew.-% bis 14 Gew.-% K₂O,
- 2 Gew.-% bis 6,5 Gew.-% Na₂O,
- 0,1 Gew.-% bis 0,2 Gew.-% Li₂O,
- 0,1 Gew.-% bis 0,3 Gew.-% Sb₂O₃,
- 0,1 Gew.-% bis 0,3 Gew.-% BaO,
- 0,6 Gew.-% bis 1,0 Gew.-% CaO,
- 0,1 Gew.-% bis 0,3 Gew.-% F.

3. Glaskeramik nach Anspruch 1 oder Anspruch 2, **dadurch gekennzeichnet, daß** ca. 5 % bis ca. 35 %, insbesondere ca. 5 % bis ca. 20 % Kristalle der ersten Gruppe vorhanden sind.

4. Glaskeramik nach einem der vorhergehenden Ansprüche, **dadurch gekennzeichnet, daß** die Kristalle der ersten Gruppe Korngrößen < 0,5 µm, vorzugsweise < 0,3 µm, aufweisen.

5. Glaskeramik nach einem der vorhergehenden Ansprüche, **dadurch gekennzeichnet, daß** die Kristalle der zweiten Gruppe Korngrößen von 1 µm bis 7 µm aufweisen.

6. Glaskeramik nach einem der vorhergehenden Ansprüche, **gekennzeichnet durch** folgende Korngrößenverteilung der Leucitkristalle in der Glasphase:
- ca. 5 % bis ca. 50 % Kristalle der ersten Gruppe,
- bis zu ca. 0,5 % Kristalle mit Korngrößen > 7 µm, und
- dem Rest, bezogen auf 100 %, Kristalle mit Korngrößen von 1 µm bis 7 µm.

7. Glaskeramik nach einem der vorhergehenden Ansprüche, **dadurch gekennzeichnet, daß** die Kristallphase im wesentlichen frei von Rissen ist.

8. Glaskeramik nach einem der vorhergehenden Ansprüche, **dadurch gekennzeichnet, daß** die Glaskeramik einen Wärmeausdehnungskoeffizienten (WAK-Wert 25 °C bis 500 °C) von 11 bis 16,5 x 10^{-6/}K und eine Brenntemperatur zwischen 700 °C und 950 °C aufweist.

9. Verfahren zur Herstellung der Glaskeramik nach einem der vorhergehenden Ansprüche, **dadurch gekennzeichnet, daß** die Leucitkristalle mit der entsprechenden Korngrößenverteilung und Glaspartikel miteinander vermischt werden, und die so erhaltene Mischung einer Wärmebehandlung bei Temperaturen zwischen 700 °C und 1.100 °C unterzogen wird.

10. Verfahren nach Anspruch 9, **dadurch gekennzeichnet, daß** die Wärmebehandlung bei Temperaturen zwischen 850 °C und 1.050 °C, vorzugsweise bei ca. 1.000 °C, durchgeführt wird.

11. Verfahren nach Anspruch 9 oder Anspruch 10, **dadurch gekennzeichnet, daß** die Wärmebehandlung zwischen 10 min. und 2 Stunden, vorzugsweise zwischen 30 min. und 1,5 Stunden, insbesondere ca. 1 Stunde, durchgeführt wird.

12. Verfahren nach einem der Ansprüche 9 bis 11, **dadurch gekennzeichnet, daß** die Leucitkristalle wie folgt hergestellt werden:
- stöchiometrisches Einwiegen der entsprechenden Komponenten, vorzugsweise K₂O, Al₂O₃ und SiO₂,
- Erschmelzen der so erhaltenen Mischung bei Temperaturen zwischen 1.400 °C und 1.600 °C,
- Wärmebehandlung des erhaltenen Produkts, vorzugsweise bei einer Temperatur von ca. 1.000 °C über einen Zeitraum von 1 Stunde, und
- Zerkleinern des wärmebehandelten Produkts auf die gewünschte Korngrößenverteilung, vorzugsweise durch mindestens einen Mahlvorgang.

13. Verwendung der Glaskeramik nach einem der Ansprüche 1 bis 8 für dentale Zwecke, insbesondere als Dentalmaterial.

14. Verwendung nach Anspruch 13 zur Verblendung von Zahnersatz, insbesondere für metallkeramischen Zahnersatz.

15. Zahnersatz, insbesondere metallkeramischer Zahnersatz, **dadurch gekennzeichnet, daß** er eine Glaskeramik nach einem der Ansprüche 1 bis 8 aufweist, insbesondere mit einer solchen Glaskeramik beschichtet oder verblendet ist.

## Claims

1. Glass-ceramic comprising a continuous glass phase and a crystal phase, comprising tetragonal leucite, wherein the glass phase is free of cracks and the crystal phase comprising leucite crystals is distributed essentially homogeneously in the glass phase and has the following particle size distribution:
- from about 5% to about 50% of a first group of crystals having particle sizes of < 1 µm and
- from about 50% to about 95% of a second group of crystals having particle sizes of ≥ 1 µm,
where up to about 1% of the crystals of the second group have particle sizes of > 7 µm and the balance to 100% has particle sizes in the range from 1 µm to 7 µm,
and where the glass-ceramic comprises the following components:
- from 60% by weight to 70% by weight of SiO₂,
- from 10% by weight to 15% by weight of Al₂O₃,
- from 10% by weight to 15% by weight of K₂O,
- from 2% by weight to 7% by weight of Na₂O,
- from 0% by weight to 0.3% by weight of Li₂O,
- from 0.1% by weight to 0.5% by weight of Sb₂O₃,
- from 0.1% by weight to 0.5% by weight of BaO,
- from 0.5% by weight to 1.0% by weight of CaO,
- from 0.1% by weight to 0.4% by weight of F.

2. Glass-ceramic according to Claim 1, **characterized in that** it comprises the following components:
- from 63% by weight to 67% by weight of SiO₂,
- from 12% by weight to 15% by weight of Al₂O₃,
- from 10% by weight to 14% by weight of K₂O,
- from 2% by weight to 6.5% by weight of Na₂O,
- from 0.1% by weight to 0.2% by weight of Li₂O,
- from 0.1% by weight to 0.3% by weight of Sb₂O₃,
- from 0.1% by weight to 0.3% by weight of BaO,
- from 0.6% by weight to 1.0% by weight of CaO,
- from 0.1% by weight to 0.3% by weight of F.

3. Glass-ceramic according to Claim 1 or Claim 2, **characterized in that** from about 5% to about 35%, in particular from about 5% to about 20%, of crystals of the first group are present.

4. Glass-ceramic according to any of the preceding claims, **characterized in that** the crystals of the first group have particle sizes of < 0.5 µm, preferably < 0.3 µm.

5. Glass-ceramic according to any of the preceding claims, **characterized in that** the crystals of the second group have particle sizes of from 1 µm to 7 µm.

6. Glass-ceramic according to any of the preceding claims, **characterized by** the following particle size distribution of the leucite crystals in the glass phase:
- from about 5% to about 50% of crystals of the first group,
- up to about 0.5% of crystals having particle sizes of > 7 µm, and
- the balance, based on 100%, of crystals having particle sizes of from 1 µm to 7 µm.

7. Glass-ceramic according to any of the preceding claims, **characterized in that** the crystal phase is essentially free of cracks.

8. Glass-ceramic according to any of the preceding claims, **characterized in that** the glass-ceramic has a coefficient of thermal expansion (CTE at from 25°C to 500°C) of from 11 to 16.5 x 10⁻⁶/K and a firing temperature of from 700°C to 950°C.

9. Process for producing the glass-ceramic according to any of the preceding claims, **characterized in that** the leucite crystals having the appropriate particle size distribution and glass particles are mixed with one another, and the resulting mixture is subjected to a heat treatment at temperatures in the range from 700°C to 1100°C.

10. Process according to Claim 9, **characterized in that** the heat treatment is carried out at temperatures in the range from 850°C to 1050°C, preferably at about 1000°C.

11. Process according to Claim 9 or Claim 10, **characterized in that** the heat treatment is carried out for from 10 minutes to 2 hours, preferably from 30 minutes to 1.5 hours, in particular about 1 hour.

12. Process according to any of Claims 9 to 11, **characterized in that** the leucite crystals are prepared as follows:
- weighing out stoichiometric amounts of the components for the leucite, preferably K₂O, Al₂O₃ and SiO₂,
- melting the mixture obtained at temperatures of from 1400°C to 1600°C,
- heat-treating the product obtained, preferably at a temperature of about 1000°C for a period of 1 hour, and
- comminuting the heat-treated product to the desired particle size distribution, preferably by means of at least one milling step.

13. Use of the glass-ceramic according to any of Claims 1 to 8 for dental purposes, in particular as dental material.

14. Use according to Claim 13 for facing tooth replacement, in particular for metal-ceramic tooth replacement.

15. Tooth replacement, in particular metal-ceramic tooth replacement, **characterized in that** it comprises a glass-ceramic according to any of Claims 1 to 8, in particular is coated or faced with such a glass-ceramic.

## Revendications

1. Vitrocéramique présentant une phase vitreuse continue et une phase cristalline en leucite tétragonale, la phase vitreuse étant exempte de fissures et la phase cristalline de cristaux de leucite étant répartie de manière essentiellement homogène dans la phase vitreuse et présentant la distribution granulométrique suivants :
- environ 5 % à environ 50 % d'un premier groupe de cristaux se présentent en grains d'une taille < 1 µm et
- environ 50 % à environ 95 % d'un deuxième groupe de cristaux se présentent en grains d'une taille ≥ 1 µm,
jusque 1 % des cristaux du deuxième groupe se présentant en grains d'une taille > 7 µm et le solde par rapport à 100 % en grains d'une taille comprise entre 1 µm et 7 µm,
la vitrocéramique contenant les composants suivantes :
- 60 % en poids à 70 % en poids de SiO₂,
- 10 % en poids à 15 % en poids de Al₂O₃,
- 10 % en poids à 15 % en poids de K₂O,
- 2 % en poids à 7 % en poids de Na₂O,
- 0 % en poids à 0,3 % en poids de Li₂O,
- 0,1 % en poids à 0,5 % en poids de Sb₂O₃,
- 0,1 % en poids à 0,5 % en poids de BaO,
- 0,5 % en poids à 1,0 % en poids de CaO,
- 0,1 % en poids à 0,4 % en poids de F.

2. Vitrocéramique selon la revendication 1, **caractérisée en ce qu'**elle contient les composants suivantes :
- 63 % en poids à 67 % en poids de SiO₂,
- 12 % en poids à 15 % en poids de Al₂O₃,
- 10 % en poids à 14 % en poids de K₂O,
- 2 % en poids à 6,5 % en poids de Na₂O,
- 0,1 % en poids à 0,2 % en poids de Li₂O,
- 0,1 % en poids à 0,3 % en poids de Sb₂O₃,
- 0,1 % en poids à 0,3 % en poids de BaO,
- 0,6 % en poids à 1,0 % en poids de CaO,
- 0,1 % en poids à 0,3 % en poids de F.

3. Vitrocéramique selon la revendication 1 ou la revendication 2, **caractérisée en ce qu'**environ 5 % à environ 35 % et en particulier environ 5 % à environ 20 % des cristaux font partie du premier groupe.

4. Vitrocéramique selon l'une des revendications précédentes, **caractérisée en ce que** les cristaux du premier groupe se présentent en grains d'une taille < 0,5 µm et de préférence < 0,3 µm.

5. Vitrocéramique selon l'une des revendications précédentes, **caractérisée en ce que** les cristaux du deuxième groupe se présentent en grains d'une taille de 1 µm à 7 µm.

6. Vitrocéramique selon l'une des revendications précédentes, **caractérisée par** la répartition granulométrique suivante des cristaux de leucite dans la phase vitreuse :
- environ 5 % à environ 50 % de cristaux du premier groupe,
- jusqu'à environ 0,5 % de cristaux en grains d'une taille > 7 µm et
- le solde par rapport à 100 % étant constitué de cristaux en grains d'une taille de 1 µm à 7 µm.

7. Vitrocéramique selon l'une des revendications précédentes, **caractérisée en ce que** la phase cristalline est essentiellement exempte de fissures.

8. Vitrocéramique selon l'une des revendications précédentes, **caractérisée en ce que** la vitrocéramique présente un coefficient de dilatation thermique (valeur WAK de 25°C à 500°C) de 11 à 16,5 x 10⁻⁶/K et une température de cuisson comprise entre 700°C et 950°C.

9. Procédé de fabrication de la vitrocéramique selon l'une des revendications précédentes, **caractérisé en ce que** les cristaux de leucite qui présentent la distribution granulométrique appropriée et des particules de verre sont mélangés les uns avec les autres et **en ce que** le mélange ainsi obtenu subit un traitement thermique à une température comprise entre 700°C et 1 100°C.

10. Procédé selon la revendication 9, **caractérisé en ce que** le traitement thermique est réalisé à une température comprise entre 850°C et 1 050°C et de préférence à environ 1 000°C.

11. Procédé selon la revendication 9 ou la revendication 10, **caractérisé en ce que** le traitement thermique est conduit pendant 10 minutes à 2 heures, de préférence pendant 30 minutes à 1,5 heures et en particulier pendant environ 1 heure.

12. Procédé selon l'une des revendications 9 à 11, **caractérisé en ce que** les cristaux de leucite sont préparés de la façon suivants :
- pesée stoechiométrique des composants appropriés, de préférence K₂O, Al₂O₃ et SiO₂,
- fusion du mélange ainsi obtenu à une température comprise entre 1 400°C et 1 600°C,
- traitement thermique pendant une durée de 1 heure du produit obtenu, de préférence à une température d'environ 1 000°C, et
- broyage du produit traité thermiquement jusqu'à la distribution granulométrique souhaitée, de préférence à l'aide d'au moins une opération de broyage.

13. Utilisation de la vitrocéramique selon l'une des revendications 1 à 8 dans des applications dentaires, en particulier comme matériau dentaire.

14. Utilisation selon la revendication 13 pour formuler des prothèses dentaires et en particulier des prothèses dentaires métalliques-céramiques.

15. Prothèse dentaire, en particulier prothèse dentaire métallique-céramique, **caractérisée en ce qu'**elle présente une vitrocéramique selon l'une des revendications 1 à 8, et en particulier est revêtue ou enduite avec une telle vitrocéramique.
